# EUROPÄISCHE PATENTSCHRIFT

(11) **EP 3 312 842 B1**
(45) Veröffentlichungstag und Bekanntmachung des Hinweises auf die Patenterteilung: **19.02.2020**
(21) Anmeldenummer: 17203435.7
(22) Anmeldetag: 24.11.2017
(51) Int. Cl.: G16H 50/30, G01C 21/34, G16H 50/80

(54) **VERFAHREN ZUM BEREITSTELLEN EINER EXPOSITIONSANGABE ÜBER EINE BELASTUNG EINER PERSON MIT LUFTSCHADSTOFFEN**
METHOD FOR PREPARING AN INDICATION OF THE EXPOSURE OF A PERSON TO TOXIC SUBSTANCES IN THE AIR
PROCÉDÉ DE FOURNITURE DE DONNÉES RELATIVES À L'EXPOSITION D'UNE PERSONNE À DES POLLUANTS ATMOSPHÉRIQUES

(43) Veröffentlichungstag der Anmeldung: 25.04.2018
(73) Patentinhaber: Carl Freudenberg KG, 69469 Weinheim (DE)
(72) Erfinder: HAEFNER, Uwe, 77694 Kehl (DE); KOEHLER, Oliver, 69469 Weinheim (DE); STAHL, Ulrich, 69514 Laudenbach (DE); OELSNER, Alexander, 68309 Mannheim (DE)

(56) Entgegenhaltungen:
- EP-A1- 3 115 745
- WO-A1-2017/114710
- GB-A- 2 539 449
- US-A1- 2006 206 576
- US-A1- 2013 080 053
- US-A1- 2013 298 642
- US-A1- 2016 117 372

## Beschreibung

### Technisches Gebiet

Die Erfindung betrifft Maßnahmen zum Bereitstellen einer Angabe über eine Schadstoffexposition eines Benutzers durch Umgebungsluft während eines längeren Zeitraums zur möglichst lückenlosen Erfassung einer Schadstoffexposition eines Benutzers durch Umgebungsluft mit variierender Luftqualität.

### Technischer Hintergrund

Messstationen zur Erfassung der Luftqualität sind aus dem Stand der Technik bekannt. Derartige Messstationen sind in der Lage, die Verschmutzung von Umgebungsluft mit Partikeln verschiedener Partikelgrößenklassen zu erfassen und/oder Konzentrationen von flüchtigen Schadstoffe, sogenannte VOCs (volatile organic compounds), in der Luft zu messen. Diese Information wird zentral gesammelt, und man kann bei einem Netz aus verteilten Messstationen eine geographische Schadstoffkonzentrationsverteilung für verschiedene Schadstoffkategorien ermitteln.

Bisherige Systeme ermöglichen somit, die lokale Luftqualität bereitzustellen, um so einem Benutzer Auskunft über die Schädlichkeit der eingeatmeten Luft geben zu können. Üblicherweise ist ein Einatmen von Luft minderer Luftqualität nicht unmittelbar gesundheitsschädlich, sondern hängt im Wesentlichen von der Expositionsdauer des Benutzers im Bereich minderer Luftqualität ab. Mindere Luftqualität liegt bei einer bezogen auf einen Referenzwert hohen Schadstoffkonzentration in einer oder in mehreren Schadstoffkategorien vor. Um eine Auskunft über eine Belastung eines Benutzers angeben zu können, ist es daher notwendig, eine kumulierte Angabe über die Belastung eines Benutzers durch das Atmen von Luft minderer Luftqualität bereitzustellen. WO2017/114710 wird als nächstliegender Stand der Technik betrachtet und offenbart eine GPS gestütztes, solitäres kumulatives Luftschadstoff-Ermittlungssystem; jedoch ohne Fahrzeugkontext. GB2539449 und US2013/080053 befassen sich auf allgemeinerer Ebene mit Luftschadstoffermittlung.

Hierbei besteht eine Schwierigkeit darin, die Luftqualität der Umgebungsluft, die der Benutzer einatmet, kontinuierlich zu berücksichtigen.

Es ist Aufgabe der vorliegenden Erfindung, verbesserte Möglichkeiten bereitzustellen, einem Benutzer eine kumulierte Luftschadstoffexposition anzugeben, den dieser durch das Einatmen von Luft unterschiedlicher Luftqualitäten hat.

### Offenbarung der Erfindung

Diese Aufgabe wird durch das Verfahren zum Ermitteln einer Exposition eines Benutzers mit Luftschadstoffen während eines längeren Zeitraums gemäß Anspruch 1 sowie durch das System zur Ermittlung einer Exposition eines Benutzers mit Luftschadstoffen gemäß dem nebengeordneten Anspruch gelöst.

Weitere Ausgestaltungen sind in den abhängigen Ansprüchen angegeben.

Gemäß einem ersten Aspekt ist ein Verfahren zum Ermitteln einer Exposition eines Benutzers mit Luftschadstoffen während eines längeren Zeitraums vorgesehen, mit folgenden Schritten:
- Bereitstellen eines zeitlichen Bewegungsprofils;
- Abrufen von zeitbezogenen und ortsbezogenen Luftqualitätsinformationen entsprechend/abhängig von dem bereitgestellten Bewegungsprofil, wobei die Luftqualitätsinformationen einen oder mehrere zeitliche Verläufe von Maßangaben für Luftschadstoffe einer oder mehreren Schadstoffkategorien angeben;
- Kumulieren der Maßangaben für die eine oder die mehreren Schadstoffkategorien entsprechend einem Zeitraster und abhängig von dem jeweiligen Ort des Benutzers;
- Bereitstellen der einen oder der mehreren kumulierten Maßangaben als Expositionsangabe eines Benutzers mit Luftschadstoffen.

Eine Idee des obigen Verfahrens besteht darin, Angaben über die Luftqualität abhängig von einem Bewegungsprofil eines Benutzers zu sammeln und daraus eine kontinuierliche kumulierte Exposition des Benutzers mit Luftschadstoffen als Expositionsangabe bereitzustellen. Dies ermöglicht einem Benutzer, zu erkennen, in welchem Maße der Benutzer Umgebungsluft minderer Luftqualität ausgesetzt war und dadurch seinen Belastungszustand erkennen. Da sich die Belastung des menschlichen Körpers aus der Schadstoffmenge in der eingeatmeten Umgebungsluft und der jeweiligen Einwirkungszeit bestimmt, kann eine Expositionsangabe die Auswirkungen zurückliegender Belastungen bemessen. Die Expositionsangabe ermöglicht dem Benutzer dann, zu entscheiden, Bereiche minderer Luftqualität künftig zu meiden.

Das zeitliche Bewegungsprofil entspricht einer Angabe über den zeitlichen Verlauf einer geografischen Position des Benutzers.
Dabei wird das Bereitstellen des zeitlichen Bewegungsprofils durch kontinuierliches Erfassen der geografischen Positionen mit Hilfe einer oder mehreren Positionslokalisationseinheiten in einem Hauptgerät und ggfs. einem oder mehreren Zusatzgeräten durchgeführt.

Eine Angabe zu einer Exposition mit Luftschadstoffen wird durch eine Kumulierung von Maßangaben eingeatmeter Luftschadstoffe, der der Benutzer ausgesetzt ist, ermittelt. Die Maßangaben der Luftschadstoffe können mithilfe eines Mobilgeräts erfasst werden, das in an sich bekannter Weise eine geographische Position des Benutzers bestimmt und eine entsprechende Information/ Maßangaben über die lokale Luftqualität abhängig von der geographischen Position von einer dezentralen Informationsquelle abruft.

Basierend auf der so erhaltenen Expositionsangabe können Funktionen ausgeführt werden, wie beispielsweise ein Warnsignal bereitgestellt werden, oder Systeme so angesteuert werden, dass die Schadstoffbelastung des betreffenden Benutzers reduziert wird.

Weiterhin können die Schadstoffkategorien Schadstoffpartikel bestimmter Größenklassen und/oder flüchtige Schadstoffe umfassen.

Die Luftqualität bestimmt sich durch die Konzentration von Schadstoffen, die in verschiedenen Schadstoffkategorien eingeteilt werden können. So können Schadstoffpartikel entsprechend ihrer Größe verschiedenen Schadstoffkategorien zugeordnet werden, wie z.B. PM1.0, PM2.5 PM10 und dergleichen. Zu jeder der Schadstoffkategorien kann die Konzentration in z.B. µg/m3 durch eine entsprechende Maßangabe angegeben werden. Gleichermaßen kann die Konzentration von flüchtigen Schadstoffen, wie z.B. der Schadstoffkategorie der Kohlenwasserstoffe, in ppm als Maßangabe angegeben werden.

Die bereitgestellten Maßangaben können für jede Schadstoffkategorie in geeigneten Zeiträumen aufaddiert werden, um so eine zeitlich kumulierte Expositionsangabe für jede der Schadstoffkategorien zur Exposition des Benutzers mit Luftschadstoffen zu erhalten.

Gemäß einer Ausführungsform kann das Abrufen von zeitbezogenen und ortsbezogenen Luftqualitätsinformationen von einer Informationsquelle regelmäßig, zu vorbestimmten Zeitpunkten, und/oder nach Aufbau einer Kommunikationsverbindung mit der Informationsquelle durchgeführt werden.

Erfindungsgemäß wird das Bereitstellen des zeitlichen Bewegungsprofils durch Plausibilisieren und Ergänzen von einem Hauptgerät und einem oder mehreren Zusatzgeräten erfassten zeitlichen Bewegungsprofilen durchgeführt.

Insbesondere im Straßenverkehr ist der Benutzer einer hohen Schadstoffbelastung durch Verkehrsabgase ausgesetzt. Jedoch kommt es gelegentlich vor, dass der Benutzer sein Mobilgerät nicht bei sich trägt und dann eine Ortsbestimmung und/oder eine Bestimmung eines Bewegungsprofils des sich in einer Fahrt befindlichen Benutzers nicht verfügbar ist. Die so entstehende zeitliche Lücke bei der Erfassung der Schadstoffexposition kann erheblich sein und die laufend zu aktualisierende Expositionsangabe verfälschen. Das obige System sieht vor, eine möglichst lückenlose Erfassung der Position des Benutzers zu ermöglichen, auch wenn dieser sein Mobilgerät nicht bei sich trägt.

Dazu wird ein Zusatzgerät zur Verfügung gestellt, das in einem Kraftfahrzeug fixierbar ist und eine von den Fahrzeugsystemen unabhängige Aufzeichnung eines Bewegungsprofils des Benutzers ermöglicht. Dieses Zusatzgerät kommuniziert durch eine geeignete Kommunikationsschnittstelle mit dem Mobilgerät, insbesondere wenn das Mobilgerät in die Nähe des Zusatzgeräts gebracht wird. Dadurch lässt sich das Bewegungsprofil des Benutzers vervollständigen und die entsprechenden zeitlichen und örtlichen Verläufe der Maßangaben über die Luftqualität zu dem entsprechenden zeitlichen Verlauf des Bewegungsprofils entlang einer geografischen Strecke abrufen. Das Zusatzgerät ermöglicht es so, die Expositionsangabe möglichst präzise zu ermitteln, indem die Wahrscheinlichkeit für das Auftreten von Erfassungslücken minimiert werden.

Es kann vorgesehen sein, dass ein Überschreiten eines vorgegebenen Grenzwerts durch die kumulierte Maßangabe einer oder mehrerer Schadstoffkategorien signalisiert wird.

Weiterhin kann die Expositionsangabe eine Summe aus mehreren gewichteten kumulierten Maßangaben enthalten. Somit können bei der Bestimmung der Expositionsangabe die Luftschadstoffe gemäß ihrer Einstufung als z.B. reizend, gesundheitsgefährdend, krebserregend unterschiedlich stark gewichtet werden, wobei stark gesundheitsgefährdende Luftschadstoffe höher gewichtet werden. Die Expositionsangabe kann dann eine Gesamtbelastung mit Luftschadstoffen bereitstellen.

### Kurzbeschreibung der Zeichnungen

Ausführungsformen werden nachfolgend anhand der beigefügten Zeichnungen näher erläutert. Es zeigen:
- Figur 1: eine schematische Darstellung eines Systems zur Erfassung einer Schadstoffexposition eines Benutzers durch Luftschadstoffe; und
- Figur 2: ein Verfahren zum Betreiben eines Mobilgeräts zur Erfassung einer Exposition von Luftschadstoffen.

### Beschreibung von Ausführungsformen

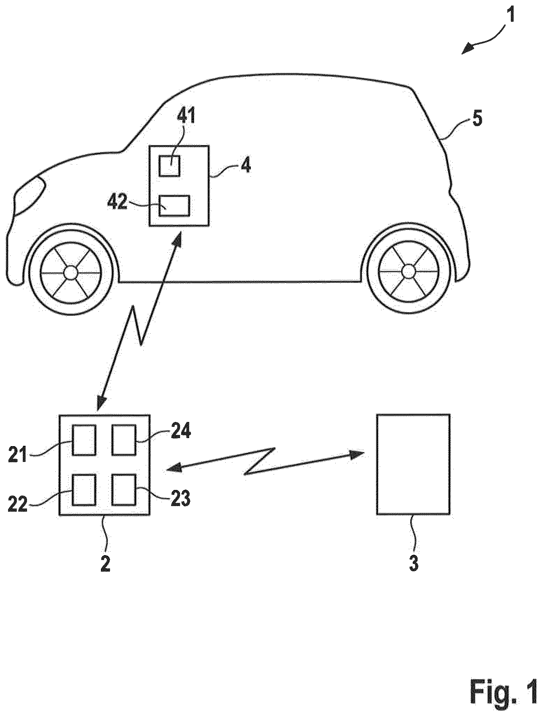

Figur 1 zeigt ein System 1 zur Erfassung einer Schadstoffexposition eines Benutzers durch Luftschadstoffe. Das System 1 umfasst ein Hauptgerät 2, das vorzugweise häufig von dem Benutzer getragen wird. Das Hauptgerät 2 kann beispielsweise ein Mobilgerät, wie beispielsweise ein Smartphone oder dergleichen sein, auf dem eine entsprechende Funktionalität mithilfe einer geeigneten Software, einer sogenannten App, implementiert ist.

Das Hauptgerät 2 umfasst eine Positionslokalisationseinheit 21, die in der Lage ist, eine geographische Position des Hauptgeräts 2 und somit des Benutzers zu erfassen. Die Positionslokalisationseinheit 21 kann beispielsweise in Form eines GPS-Empfängers oder dergleichen ausgebildet sein, die mithilfe einer satellitengestützen Ortung oder einem vergleichbaren Ortungsverfahren die geografische Position des Hauptgeräts 2 bestimmen kann. Alternativ kann die Positionslokalisationseinheit 21 auch ein Kommunikationsmodul umfassen und eine Funktion zur Ortsbestimmung durchführen, die beispielsweise den Ort des Hauptgeräts 2 durch eine Geolokalisierung mithilfe einer IP-Adresse oder durch Triangulation mithilfe von Mobilfunksendeanlagen ausführt.

Weiterhin umfasst das Hauptgerät 2 einen Mikroprozessor 22 und eine Speichereinheit 23, die zur Ausführung von Softwarecodes und Algorithmen geeignet ist. Weiterhin ist eine Kommunikationseinheit 24 vorgesehen, die es dem Hauptgerät 2 ermöglicht, mit einer externen Informationsquelle 3 eine Datenverbindung aufzubauen, um Daten zu senden und zu empfangen.

Die Informationsquelle 3 dient dazu und ist dazu ausgelegt, abhängig von einer empfangenen geographischen Position und einem Zeitstempel des Hauptgeräts 2 Angaben über eine Luftqualität in Form von Maßangaben für eine oder mehrere Schadstoffkategorien zur Verfügung zu stellen, die die Luftqualität an der geografischen Position zu dem durch den Zeitstempel angegebenen Zeitpunkt angeben. Die Maßangaben über die Luftqualität können Konzentrationsangaben von Schafstoffen einer oder mehreren Schadstoffkategorien an der geographischen Position und zu einem bestimmten Zeitpunkt bzw. Zeitbereich angeben. Die Informationsquelle 3 ermöglicht also den Abruf von Luftqualitätsinformationen für geographische Positionen und Zeitpunkte. Die Schadstoffkategorien können beispielsweise Schadstoffe von Luftpartikeln verschiedener Größenbereiche, wie z. B. PM1, PM2,5, usw. sowie Kategorien von flüchtigen Schadstoffen, sogenannten VOCs (volatile organic compounds), wie z.B. Kohlenwasserstoffe, angeben. Die Maßangaben sind den Schadstoffkategorien zugeordnet und geben in geeigneter Weise deren Konzentration an, insbesondere in ppm für flüchtige Schadstoffe und/oder µg/m3 für Partikel.

Weiterhin kann ein mobiles Zusatzgerät 4 vorgesehen sein, das z. B. in einem Kraftfahrzeug 5 vorgesehen ist. Das Zusatzgerät 4 kann ebenfalls eine Positionslokalisationseinheit 41 zur Bestimmung der geographischen Position des Hilfsgeräts 4 aufweisen und in der Lage sein, eine Kommunikationsverbindung über eine geeignete Kommunikationseinrichtung 42, wie z.B. ein Wifi-Modul, Bluetooth-Modul, NFC-Modul oder dergleichen mit dem Hauptgerät 2 aufzubauen.

Das Zusatzgerät 4 weist eine Steuereinheit 43 und einen Speicher 44 auf. Die Steuereinheit 43 ist ausgebildet, um mithilfe der Positionslokalisationseinheit 41 bei einer Bewegung des Kraftfahrzeugs 5 diese zeitbezogen (mit Zeitstempel) aufzeichnet und in dem Speicher 44 als Bewegungsprofil speichert.

Das Hauptgerät 2 kann nun durch die Kommunikationsverbindung mit dem Zusatzgerät 4 das Bewegungsprofil abrufen und das in dem Hauptgerät 2 aufgezeichnete Bewegungsprofil entsprechend ergänzen. Auf diese Weise ist es möglich, zum einen das in dem Hauptgerät 2 aufgezeichnete Bewegungsprofil zu plausibilisieren, wenn der Benutzer das Mobilgerät während einer Fahrt mit sich trägt, oder das Bewegungsprofil zu ergänzen, wenn der Benutzer während einer Fahrt das Mobilgerät nicht bei sich trägt, weil dieser es beispielsweise vergessen hat. Wird von dem Hauptgerät 2 nachträglich ein Bewegungsprofil empfangen, das das Hauptgerät nicht erfasst hat, so können für die Vergangenheit die Angaben über die Luftqualität von der Informationsquelle 3 abgerufen werden und die Schadstoffexposition entsprechend aktualisiert werden.

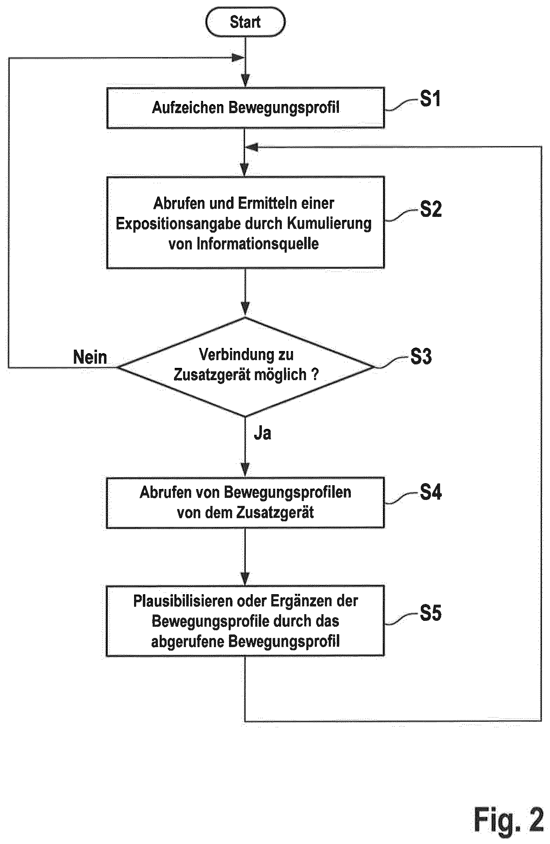

In Figur 2 ist ein Flussdiagramm zur Veranschaulichung eines Verfahrens zum Überwachen einer Schadstoffbelastung eines Benutzers anhand eines Flussdiagramms dargestellt.

In Schritt S1 wird durch das Hauptgerät 2 ein Bewegungsprofil aufgezeichnet und dazu die durch die Positionslokalisationseinheit 21 erfassten geographischen Positionen zusammen mit einem entsprechenden Zeitstempel erfasst.

Kontinuierlich oder zu regelmäßigen oder vorbestimmten Zeitpunkten werden in Schritt S2 anhand der Bewegungsprofile Luftqualitätsinformationen von der Informationsquelle 3 abgerufen und die entsprechenden Schadstoffkonzentrationen der einzelnen Schadstoffkategorien bezüglich eines Zeitinkrements kumuliert. Das Zeitinkrement kann beispielsweise 1 Stunde bis 3 Stunden, 6 Stunden oder einen Tag umfassen. Insbesondere kann der Abruf der zeit- und ortsbezogenen Luftqualitätsinformationen zu einem Zeitpunkt erfolgen, zu dem eine Kommunikationsverbindung mit der Informationsquelle 3 aufgebaut werden kann, insbesondere wenn eine Internetverbindung zwischen dem Hauptgerät 2 und der Informationsquelle 3 besteht.

In einem nachfolgenden Schritt S3 wird abgefragt, ob eine Kommunikationsverbindung zu einem dem Hauptgerät 2 bekannten Zusatzgerät 4 aufgebaut werden kann. Kann eine Verbindung aufgebaut werden (Alternative: Ja), so wird das Verfahren mit Schritt S4 fortgesetzt, anderenfalls (Alternative: Nein) wird zu Schritt S1 zurückgesprungen.

In Schritt S4 werden Informationen über Bewegungsprofile des Zusatzgeräts 4 abgefragt die auf gleiche Weise wie in dem Hauptgerät 2 mit Zeitstempel erfasst worden sind.

In Schritt S5 wird das Bewegungsprofil in dem Hauptgerät 2 zur Plausibilisierung erfasster Bewegungsprofile verwendet, oder im Hauptgerät 2 befindliche Bewegungsprofile werden durch die durch das Zusatzgerät 4 erfassten Bewegungsprofile ergänzt. Anschließend werden für die neu erfassten Bewegungsprofile die entsprechenden Luftqualitätsinformationen orts- und zeitbezogen abgerufen. Eine Plausibilisierung kann beispielsweise erfolgen, indem eine letzte erfasste geografische Position mit einer aktuellen geografischen Position verglichen wird. Ist der geografische Abstand zu groß, um in der verstrichenen Zeit mit einem Fahrzeug zurückgelegt worden zu sein, ergibt sich ein negatives Plausibilisierungsergebnis bzw. es fehlen Daten für diese "Erfassungslücke". Der Benutzer kann die Erfassungslücke nachträglich manuell selbst schließen, indem er von der App eine Abfrage bekommt, welche dem Nutzer die Möglichkeit bietet Daten von Orten inklusive Zeitfenstern einzufügen. Diese historischen Daten können ebenfalls von externen Quellen bezogen werden.

Das Zusatzgerät 4 kann in dem Kraftfahrzeug 5 fest montiert oder herausnehmbar ausgebildet sein. Insbesondere kann es in Form einer Halterung für ein Mobiltelefon (Hauptgerät) ausgebildet sein, wobei bei Einsetzen des Mobiltelefons automatisch eine Kommunikationsverbindung mit dem Zusatzgerät 4 erstellt wird, um das durch das Zusatzgerät 4 erfasste Bewegungsprofil an das Hauptgerät 2 zu übertragen.

## Patentansprüche

1. Verfahren zum Ermitteln einer Expositionsangabe als kontinuierliche kumulierte Exposition eines Benutzers mit Luftschadstoffen während eines längeren Zeitraums, mit folgenden Schritten:
- Bereitstellen (S1) eines zeitlichen Bewegungsprofils als Angabe über den zeitlichen Verlauf einer geografischen Position des Benutzers während einer Fahrt, wobei das Bereitstellen des zeitlichen Bewegungsprofils durch kontinuierliches Erfassen von geografischen Positionen mit Hilfe einer oder mehrere Positionslokalisationseinheiten (21) in einem als Mobilgerät ausgeführten Hauptgerät (2) und einem in einem Kraftfahrzeug fixierbaren Zusatzgerät (4) durchgeführt wird, wobei durch das Zusatzgerät eine von dem Fahrzeugsystem unabhängige Aufzeichnung eines Bewegungsprofils des Benutzers ermöglicht wird, und das Zusatzgerät über eine Kommunikationsschnittstelle mit dem Mobilgerät kommuniziert, wobei das Bereitstellen des zeitlichen Bewegungsprofils durch Plausibilisieren und Ergänzen von dem Hauptgerät (2) und dem Zusatzgerät (4) erfassten zeitlichen Bewegungsprofilen durchgeführt wird (S4, S5); wobei das in dem Mobilgerät (2) aufgezeichnete Bewegungsprofil plausibilisiert wird, wenn der Benutzer das Mobilgerät während einer Fahrt mit sich trägt, und zur Plausibilisierung eine letzte erfasste geografische Position mit einer aktuellen geografischen Position verglichen werden kann ,wobei, wenn der geografische Abstand zu groß ist, um in der verstrichenen Zeit mit einem Fahrzeug zurückgelegt worden zu sein, eine Erfassungslücke diagnostiziert wird, die vom Benutzer manuell geschlossen werden kann, und wobei das in dem Mobilgerät (2) aufgezeichnete Bewegungsprofil ergänzt wird und ein vervollständigtes zeitliches Bewegungsprofil entsteht, wenn der Benutzer das Mobilgerät während einer Fahrt nicht mit sich trägt und zur Ergänzung das Bewegungsprofil des Zusatzgerätes (4) verwendet wird
- Abrufen (S2) von zeitbezogenen und ortsbezogenen Luftqualitätsinformationen von einer Informationsquelle (3) abhängig von dem bereitgestellten zeitlichen Bewegungsprofil, wobei die Luftqualitätsinformationen einen oder mehrere zeitliche Verläufe von Maßangaben für Luftschadstoffe einer oder mehreren Schadstoffkategorien für mehrere Orte angeben;
- Kumulieren (S2) der Maßangaben für die eine oder die mehreren Schadstoffkategorien entsprechend einem Zeitraster und abhängig von dem jeweiligen Ort des Benutzers; und
- Bereitstellen der einen oder der mehreren kumulierten Maßangaben als Expositionsangabe eines Benutzers mit Luftschadstoffen, wobei die Expositionsangabe die Auswirkung zurückliegender Belastung bemisst, wobei die Expositionsangabe entsprechend aktualisiert wird indem für die Vergangenheit die Luftqualitätsinformationen von der Informationsquelle (3) abgerufen werden, wenn von dem Mobilgerät (2) nachträglich ein Bewegungsprofil empfangen wird, das das Mobilgerät (2) nicht erfasst hat, wobei das Bewegungsprofil vom Zusatzgerät (4) abgerufen wird.

2. Verfahren nach Anspruch 1, wobei die Schadstoffkategorien Schadstoffpartikel bestimmter Größenklassen und/oder flüchtige Schadstoffe umfassen.

3. Verfahren nach Anspruch 1 oder 2, wobei das Abrufen von zeitbezogenen und ortsbezogenen Luftqualitätsinformationen von einer Informationsquelle (3) regelmäßig, zu vorbestimmten Zeitpunkten, und/oder nach Aufbau einer Kommunikationsverbindung mit der Informationsquelle (3) durchgeführt wird.

4. Verfahren nach einem der Ansprüche 1 bis 3, wobei ein Überschreiten eines vorgegebenen Grenzwerts durch die kumulierte Maßangabe einer oder mehrerer Schadstoffkategorien, insbesondere optisch oder akustisch, signalisiert wird.

5. Verfahren nach einem der Ansprüche 1 bis 4, wobei die Expositionsangabe mit einer Summe aus mehreren gewichteten kumulierten Maßangaben bestimmt wird.

## Claims

1. Method for determining an exposure indication as a continuous cumulated exposure of a user to toxic substances in the air over a relatively long period of time, having the following steps:
- providing (S1) a time motion profile as an indication over the course of time of a geographical position of the user during a journey, wherein the provision of the time motion profile is carried out by continuously acquiring geographical positions using one or more position localization units (21) in a main device (2) which is embodied as a mobile device, and an additional device (4) which can be secured in a motor vehicle, wherein the additional device makes possible recording of a motion profile of the user which is independent of the vehicle system, and the additional device communicates with the mobile device via a communication interface, wherein the provision of the time motion profile is carried out (S4, S5) by checking plausibility and adding to time motion profiles which are acquired by the main device (2) and the additional device (4); wherein the plausibility of the motion profile which is recorded in the mobile device (2) is checked if the user is carrying the mobile device on his person during a journey, and in order to check the plausibility a last acquired geographical position can be compared with a current geographical position, wherein if the geographical distance is too large to be covered with a vehicle in the time which has passed, an acquisition gap is diagnosed, which can be closed manually by the user, and wherein the motion profile which is recorded in the mobile device (2) is added to and a completed time motion profile is produced if the user is not carrying the mobile device on his person during a journey and is used to add to the motion profile of the additional device (4),
- retrieving (S2) time-related and location-related air quality information items from an information source (3) as a function of the provided time motion profile, wherein the air quality information items indicate one or more temporal progressions of dimension indications for toxic substances in the air of one or more toxic substance categories for a plurality of locations;
- cumulating (S2) the dimension indications for the one or more toxic substance categories in accordance with a time grid and as a function of the respective location of the user; and
- providing the one or more cumulated dimension indications as an exposure indication of a user to toxic substances in the air, wherein the exposure indication rates the effect of a preceding load, wherein the exposure indication is correspondingly updated by retrieving the air quality information items from the information source (3) for the past if a motion profile which the mobile device (2) has not acquired is subsequently received by the mobile device (2), wherein the motion profile is retrieved by the additional device (4).

2. Method according to Claim 1, wherein the toxic substance categories comprise toxic substance particles of specific size classes and/or volatile toxic substances.

3. Method according to Claim 1 or 2, wherein the retrieval of time-related and location-related air quality information items of an information source (3) is carried out regularly, at predetermined times and/or after the setup of a communication link to the information source (3).

4. Method according to one of Claims 1 to 3, wherein an exceeding of a predefined limiting value by the cumulated dimension indication of one or more toxic substance categories is signalled, in particular optically or acoustically.

5. Method according to one of Claims 1 to 4, wherein the exposure indication is determined with a sum of a plurality of weighted cumulated dimension indications.

## Revendications

1. Procédé de détermination d'une indication d'exposition en tant qu'exposition cumulée continue d'un utilisateur à des polluants atmosphériques pendant une période prolongée, comprenant les étapes suivantes :
- fourniture (S1) d'un profil de mouvement temporel comme indication de l'évolution dans le temps d'une position géographique de l'utilisateur pendant un déplacement, la fourniture du profil de mouvement temporel étant effectuée par l'acquisition continue de positions géographiques à l'aide d'une ou plusieurs unités de localisation de position (21) dans un appareil principal (2) réalisé sous la forme d'un appareil mobile et un appareil supplémentaire (4) qui peut être calé dans un véhicule automobile, un enregistrement indépendant du système de véhicule d'un profil de mouvement de l'utilisateur étant rendu possible par l'appareil supplémentaire, et l'appareil supplémentaire communiquant avec l'appareil mobile par le biais d'une interface de communication, la fourniture du profil de mouvement temporel étant effectuée (S4, S5) en vérifiant la plausibilité et en complétant les profils de mouvement temporels acquis par l'appareil principal (2) et l'appareil supplémentaire (4) ; le profil de mouvement enregistré dans l'appareil mobile (2) étant considéré plausible lorsque l'utilisateur emporte l'appareil mobile avec lui pendant un déplacement et pour vérifier la plausibilité une comparaison d'une dernière position géographique acquise avec une position géographique actuelle pouvant être effectuée , une lacune d'acquisition qui peut être comblée manuellement par l'utilisateur étant diagnostiquée lorsque l'écart géographique est trop grand pour avoir été parcouru par un véhicule pendant le temps écoulé, et le profil de mouvement enregistré dans l'appareil mobile (2) étant complété et un profil de mouvement temporel complété étant obtenu lorsque l'utilisateur n'emporte pas l'appareil mobile avec lui pendant un déplacement et en complément, le profil de mouvement de l'appareil supplémentaire (4) étant utilisé ;
- invocation (S2) d'informations de qualité d'air référencées au temps et localisées auprès d'une source d'informations (3) en fonction du profil de mouvement temporel fourni, les informations de qualité d'air indiquant une ou plusieurs évolutions dans le temps d'indications dimensionnelles pour des polluants atmosphériques d'une ou plusieurs catégories de polluants pour plusieurs endroits ;
- cumul (S2) des indications dimensionnelles pour l'une ou les plusieurs catégories de polluants conformément à une grille temporelle et en fonction de l'endroit respectif de l'utilisateur ; et
- fourniture de l'une ou des plusieurs indications dimensionnelles cumulées comme indication d'exposition d'un utilisateur à des polluants atmosphériques, l'indication d'exposition mesurant l'effet de l'exposition écoulée, l'indication d'exposition étant actualisée en conséquence en invoquant les informations de qualité d'air du passé auprès de la source d'informations (3) lorsque l'appareil mobile (2) reçoit à postériori un profil de mouvement que l'appareil mobile (2) n'a pas acquis, le profil de mouvement étant invoqué auprès de l'appareil supplémentaire (4).

2. Procédé selon la revendication 1, les catégories de polluants comprenant des particules de polluant de classes de taille définies et/ou des polluants volatils.

3. Procédé selon la revendication 1 ou 2, l'invocation d'informations de qualité d'air référencées au temps et localisées auprès d'une source d'informations (3) étant effectuée régulièrement, à des instants prédéfinis et/ou après établissement d'une liaison de communication avec la source d'informations (3).

4. Procédé selon l'une des revendications 1 à 3, un dépassement d'une valeur limite prédéfinie par l'indication dimensionnelle cumulée d'une ou plusieurs catégories de polluants étant signalé, notamment visuellement ou de manière sonore.

5. Procédé selon l'une des revendications 1 à 4, l'indication d'exposition étant déterminée avec une somme de plusieurs indications dimensionnelles cumulées pondérées.
